# EUROPEAN PATENT SPECIFICATION

(11) **EP 4 516 926 B1**
(45) Date of publication and mention of the grant of the patent: **16.09.2026**
(21) Application number: 23794701.5
(22) Date of filing: 16.02.2023
(51) Int. Cl.: C12Q 1/6869

(54) **GENE SEQUENCING METHOD**
GENSEQUENZIERUNGSVERFAHREN
PROCÉDÉ DE SÉQUENÇAGE GÉNIQUE

(30) Priority: 27.04.2022 CN 202210449634
(43) Date of publication of application: 05.03.2025
(73) Proprietor: Shenzhen Salus Biomed Co., Ltd, Shenzhen, Guangdong 518107 (CN)
(72) Inventor: ZHANG, Changling, Shenzhen, Guangdong 518107 (CN); WANG, Gufeng, Shenzhen, Guangdong 518107 (CN); ZHAO, Luyang, Shenzhen, Guangdong 518107 (CN)
(74) Representative: Cabinet Chaillot
(86) International application number: PCT/CN2023/076378
(87) International publication number: WO 2023/207265

(56) References cited:
- EP-A1- 3 702 474
- WO-A2-2006/083751
- CN-A- 110 818 757
- CN-A- 112 218 640
- CN-A- 112 601 825
- CN-A- 114 958 995
- CN-A- 114 981 283
- US-A1- 2018 208 983
- US-B2- 9 175 342
- J. GUO ET AL: "Four-color DNA sequencing with 3'-O-modified nucleotide reversible terminators and chemically cleavable fluorescent dideoxynucleotides", PROCEEDINGS OF THE NATIONAL ACADEMY OF SCIENCES, vol. 105, no. 27, 1 January 2008 (2008-01-01), pages 9145 - 9150, XP055013741, ISSN: 0027-8424, DOI: 10.1073/pnas.0804023105
- "The Handbook of Plant Mutation Screening", 27 January 2010, WILEY-VCH VERLAG GMBH & CO. KGAA, Weinheim, Germany, ISBN: 978-3-527-32604-4, article LIN YU ET AL: "DNA Sequencing-by-Synthesis using Novel Nucleotide Analogs", pages: 319 - 338, XP055672229, DOI: 10.1002/9783527629398.ch19

## Description

### TECHNICAL FIELD

The invention belongs to the field of gene sequencing, and particularly relates to a gene sequencing method.

### BACKGROUND

Since the invention of Sanger sequencing, DNA sequencing technology has seen a history of over 40 years, during which the first-generation sequencing technology represented by the dideoxy chain termination sequencing method was developed and then the second-generation sequencing technology focused on sequencing by synthesis (SBS) emerged to overcome the defects of high cost and low throughput of the first-generation sequencing technology. The Illumina's SBS sequencing technology, as a representative one of the existing second-generation SBS sequencing technologies, identifies and distinguishes the four types of bases (adenine A, guanine G, cytosine C and thymine T) in DNA sequences by detecting fluorescence signals. Specifically, such a method uses dNTPs (dATP, dCTP, dGTP and dTTP) with fluorescent marker and blocking groups, wherein dATP, dCTP, dGTP and dTTP carry different fluorescent marker groups respectively. Due to the presence of the blocking groups, only one complementary dNTP will be added to each DNA template in case of DNA polymerization, the type of the added dNTP in this cycle can be detected by excitation with an exciting light of the corresponding wavelength band, and then the reversible blocking group and the fluorescent marker group can be removed with a suitable chemical regent to allow for the next cycle of normal chemical sequencing reaction.

At present, key raw materials that are indispensable for existing SBS-based sequencing methods are the dNTPs with reversible blocking groups and fluorescent marker groups and suitable chemical reagents capable of completely removing the reversible blocking groups and fluorescent marker groups. However, due to the complexity of the structure synthesized by the dNTPs, there is not yet a satisfying reagent that can completely remove the reversible blocking groups and fluorescent marker groups to return the dNTPs to the natural state for the next cycle of sequencing reaction. In this process, a branched chain, which is also referred to as a synthetic scar, that fails to be removed will be left on a new synthetic DNA chain after each cycle of reaction. Due to the cumulative effect, these branched chains will affect dNTP synthesis and removal efficiency more and more as the reaction progresses. As a result, the existing second-generation sequencing techniques are only suitable for sequencing of short DNA sequences, generally in the range of 100-400 bases.

Therefore, a gene sequencing method that can completely remove blocking groups and fluorescent groups is desired.

### SUMMARY

To solve at least one of the abovementioned technical problems in the prior art, the invention provides a gene sequencing method.

In one aspect, a gene sequencing method, according to claim 1, includes the following steps:
S1: hybridizing a sequencing primer onto a nucleic acid molecule to be detected to form a hybrid template strand and a primer strand;
S2: performing base pairing on a first nucleotide analog and the nucleic acid molecule to be detected, and linking the first nucleotide analog to the primer strand, wherein the first nucleotide analog has a blocking group;
S3: performing base pairing on a second nucleotide analog and the nucleic acid molecule to be detected, the second nucleotide analog forming a complex with the nucleic acid molecule to be detected and the first nucleotide analog under the action of metal ions and a polymerase, wherein the metal ions are bonded with coordinating atoms in the first nucleotide analog and coordinating atoms in the second nucleotide analog such that the second nucleotide analog is stably chelated at an end of the primer strand, and wherein the second nucleotide analog has a marker; wherein the chemical formula of the second nucleotide analog is as follows: 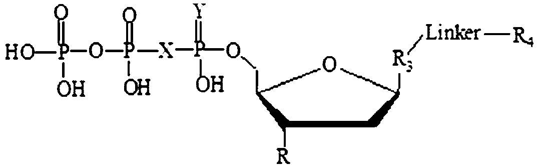
where, X and Y are each any one of oxygen (O), sulfur (S) and imino (NH), and X and Y are not both oxygen (O); R is any one of hydroxy (-OH), hydrogen (H), methoxyl (-OMe), amino (-NH₂) and sulfhydryl (-SH); R₃ is any one of A, G, C and T, Linker is a linker, and R₄ is the marker; wherein the metal ions are divalent metal ions, comprising at least one of Mg²⁺, Cu²⁺, Zn²⁺, Mn²⁺ and Ca²⁺;
S4: detecting the marker, and identifying a base in the nucleic acid molecule to be detected; and
S5: removing the blocking group and the second nucleotide analog, and repeating S2-S4 to perform a next cycle of sequencing.

According to one preferred embodiment of the invention, the invention has at least the following beneficial effects:
In the invention, a second nucleotide analog is bonded on a primer strand by forming a complex with a first nucleotide analog under the action of metal ions and a polymerase. After the metal ions are removed, the second nucleotide analog cannot be stably bonded with the first nucleotide analog anymore, such that the blocking group and marker excision requirement and difficulty are reduced significantly, and blocking groups and markers can be removed more completely without synthetic scars, thus greatly increasing the sequencing length and reducing the sequencing cost. In addition, blocking groups and markers are labelled on different nucleotides and are not located on the same nucleotide, thus greatly reducing the synthesis difficulty and improving the synthesis flexibility.

The gene sequencing method is suitable for various types of DNA sequencing, including but not limited to, genomic DNA sequencing, single-gene sequencing and multi-gene assembly sequencing.

In some embodiments of the invention, before S1, a sequencing library is prepared by extracting nucleic acid, performing fragmentation, end preparation, dA-tailing and adapter ligation on the nucleic acid, and then preparing library samples by library amplification and library quality control.

In some embodiments of the invention, after the library samples are prepared, the library samples need to be amplified.

In some embodiments of the invention, the template strand is the nucleic acid molecule to be detected.

In some embodiments of the invention, the primer strand is a strand starting from the sequencing primer and complementary with the template strand, and may be linked to the first nucleotide analog after the sequencing primer.

In some embodiments of the invention, the first nucleotide analog and the second nucleotide analog may be artificially synthesized or commercially available.

In some embodiments of the invention, the blocking group is linked to a 3'-hydroxyl of the first nucleotide analog.

In some embodiments of the invention, the blocking group comprises at least one of azido-methylene, allyl, 2-nitrobenzene methyl and azoic compounds.

In some embodiments of the invention, the chemical formula of the first nucleotide analog is as follows: 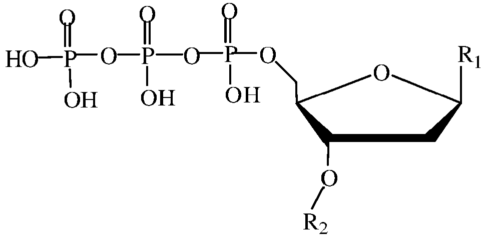 where, R₁ is any one of A, G, C and T, and R₂ is the blocking group.

In some embodiments of the invention, the marker comprises at least one of Alexa Fluor, iFluor, cyanine, ROX and derivatives thereof.

In some embodiments of the invention, the linker comprises at least one of alkyl, allyl, azido-methylene, 2-nitrobenzyl and di-sulfhydryl.

In some embodiments of the invention, a DNA polymerase is added in S2, and under the action of the DNA polymerase, the formation of a phosphodiester bond between a 5'-phosphate group of the first nucleotide analog and a 3'-hydroxyl of a last nucleotide of the primer strand is catalyzed to link the first nucleotide analog to a 3'-end of the primer strand.

In some preferred embodiments of the invention, the DNA polymerase is any one of a polymerase 9N, a taq DNA polymerase, a Vent DNA polymerase, a phi29 DNA polymerase, a Bst DNA polymerase, a Bsu DNA polymerase and a Klenow DNA polymerase.

In some preferred embodiments of the invention, the metal ions comprise at least one of Mg²⁺ and Cu²⁺.

In the presence of the divalent metal ions, the second nucleotide analog can be stably bonded to the primer strand.

Specifically, the second nucleotide analog may be specifically determined and selected according to a base, commentary with the second nucleotide analog, in the template strand.

In some embodiments of the invention, in S4, the marker is detected by observing a solid phase of a double-stranded DNA formed by the template strand and the primer strand through a fluorescence microscope or an optical system of a sequencer or by performing fluorescence detection on a solution containing the double-stranded DNA through a fluorescence detector.

In some embodiments of the invention, in S5, a buffer containing a metal chelator is added to be bonded with and remove the metal ions to as to release the second nucleotide analog from the primer strand.

In some embodiments of the invention, the metal chelator comprises at least one of ethylenediaminetetraacetic acid, ethylenediaminetetraacetate, nitrilotriacetic acid, citric acid, citrate, tartaric acid and gluconic acid.

After the metal ions are removed, the second nucleotide analog cannot be stably bonded on the 3'-end of the primer strand anymore, which is conducive to completely removing the second nucleotide analog, that is, conducive to completely removing the marker.

In some embodiments of the invention, in S5, the blocking group is removed by photocleavage or by adding an organic reagent, and the organic reagent comprises at least one of a sulfhydryl group reagent, an organic phosphine reagent and sodium hydrosulfite.

### BRIEF DESCRIPTION OF THE DRAWINGS

The invention is further described below in conjunction with accompanying drawings and embodiments. In the drawings:
FIG. 1 illustrates chemical structures of four first nucleotide analogs synthesized according to Embodiment 1 of the invention;
FIG. 2 illustrates chemical structures of four second nucleotide analogs synthesized according to Embodiment 1 of the invention;
FIG. 3 is schematic diagram of the sequencing principle according to Embodiment 1 of the invention;
FIG. 4 illustrates signal images of bases detected in a first cycle and a 100^{th} cycle according to Embodiment 1 of the invention, wherein the upper signals images correspond to the first cycle, and the lower signals images correspond to the 100^{th} cycle;
FIG. 5 illustrates Q values according to Embodiment 1 of the invention.

### DESCRIPTION OF THE EMBODIMENTS

The embodiments of the invention are described in detail below. In the description of the invention, "first" and "second", if any, are merely used for distinguishing technical features and should not be construed as indicating or implying relative importance or implicitly indicating the number or the precedence relationship of technical features referred to.

In the description of the invention, unless otherwise expressly stated, terms such as "synthesize" and "detect" should be broadly understood, and those skilled in the art can rationally determine the specific meanings of these terms in the invention in conjunction with specific contents of the technical solutions.

In the description of the invention, reference terms such as "one embodiment" and "some embodiments" are intended to indicate that specific features, structures, materials or characteristics described in conjunction with said embodiment(s) are included in at least one embodiment of the invention. Illustrative descriptions of these terms do not definitely refer to the same embodiments. In addition, the specific features, structures, materials or characteristics described here can be combined appropriately in any one or more embodiments.

Unless otherwise specially stated, all test methods used in the embodiments are conventional methods. Unless otherwise specially stated, all materials and reagents used are commercially available materials and reagents.

### Embodiment 1

In this embodiment, sequencing was performed on a human genomic library. The specific process is as follows:

### 1. Preparation of a genomic sequencing library:

(1) Extraction of genomic DNA: human genomic DNA was extracted using a rapid DNA extraction kit (TIANGEN, KG203) according to kit instructions;
(2) Fragmentation, end repair and dA-tailing and adapter ligation of the extracted human genomic DNA, library amplification and library quality control using the universal library preparation kit VAHTS^{®} Universal Plus DNA Library Prep Kit for Illumina (No.: ND617-02): fragmentation, end filling, 5'-end phosphorylation and 3'-end dA-tailing were performed on the human genomic DNA extracted in Step (1) to obtain an end repair product; a specific Illumina Adapter was added to the end repair product, and an adapter ligation product was purified; PCR enrichment was performed on the purified adapter ligation product, and then the quality of the enriched library was evaluated by length distribution detection and concentration detection, such that a library sample with a concentration of about 50nM and a length of about 100-1000 bp was obtained;
(3) Amplification of library sample: denaturation was performed on the library sample, which dilutes the library sample to a concentration of 4nM with sterile water, and library denaturation was performed according to Table 1 to obtain a library intermediate sample having a concentration of 20pM;

**Table 1**

| Components of reagent | Volume/µL |
|---|---|
| 0.2M NaOH | 5 |
| 4nM library samples | 5 |
| 200mM Tris-HCl ( pH 8.0 ) | 5 |
| Hybridization solution | 985 |
| 20pM library intermediate samples | 1000 |

Taking the 20pM library intermediate sample prepared above as a mother liquor, a library sample with a concentration required for loading was prepared according to Table 2:

**Table 2**

| Loading concentration | 1pM | 1.5pM | 3pM | 4.5pM | 6pM |
|---|---|---|---|---|---|
| Usage amount of 20pM library intermediate sample/µL | 160 | 200 | 240 | 280 | 400 |
| Usage amount of hybridization solution/µL | 1440 | 1400 | 1360 | 1320 | 1200 |

Library amplification was performed on the surface of a chip using the Miseq sequencer and its sequencing kit (Miseq Reagent Kit v3) provided by Illumina to obtain a DNA library amplification cluster, and a sequencing primer (SEQ ID NO : 1 : ACACTCTTTCCCTACACGACGCTCTTCCGATC) was added and hybridized with the DNA library amplification cluster, and after completion of the hybridization, the resulted product is pending for subsequent sequencing reaction.

### 2. Sequencing:

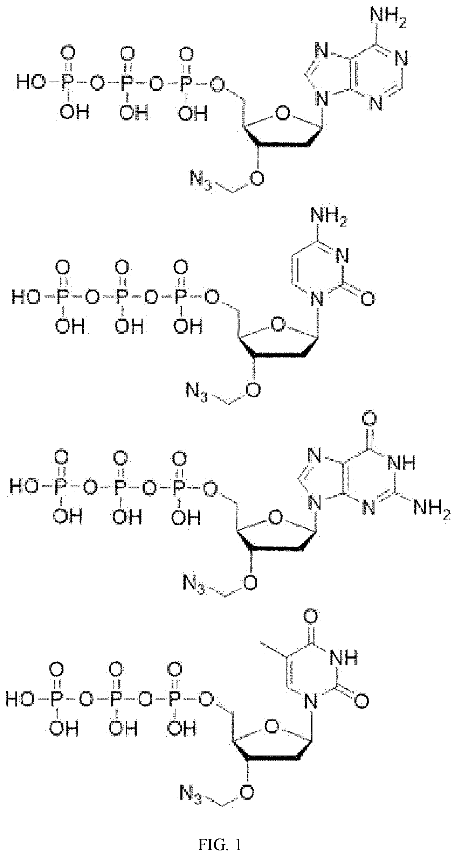

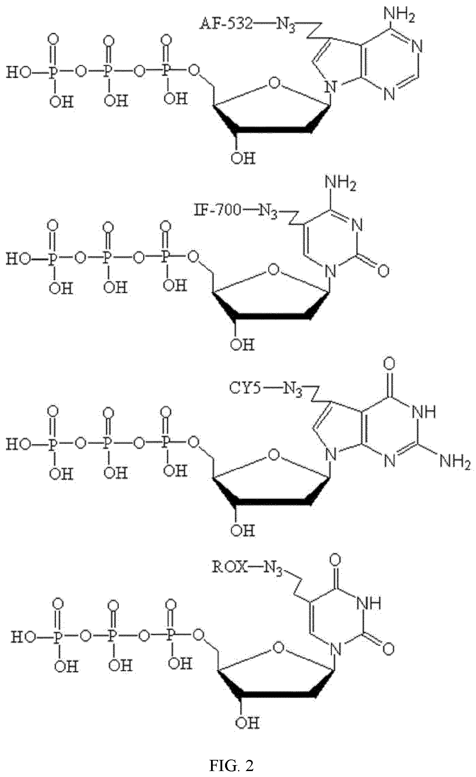

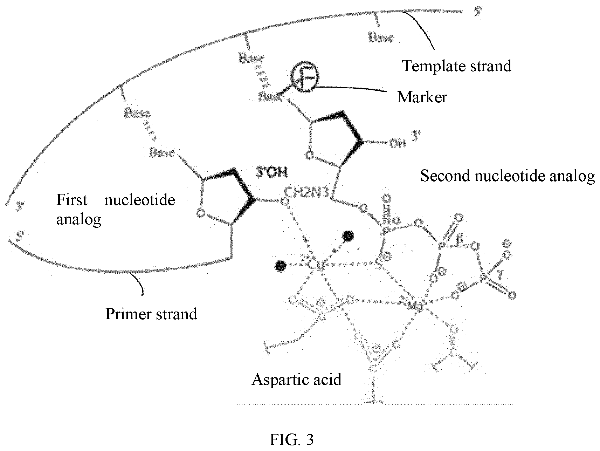

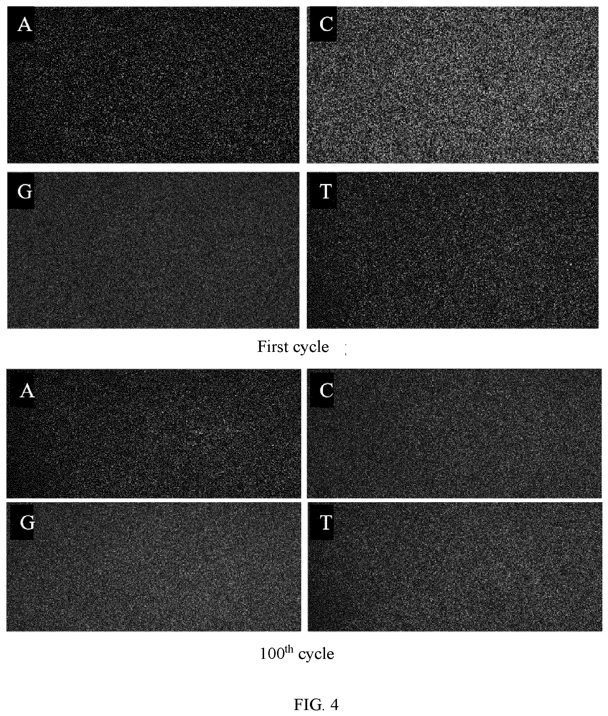

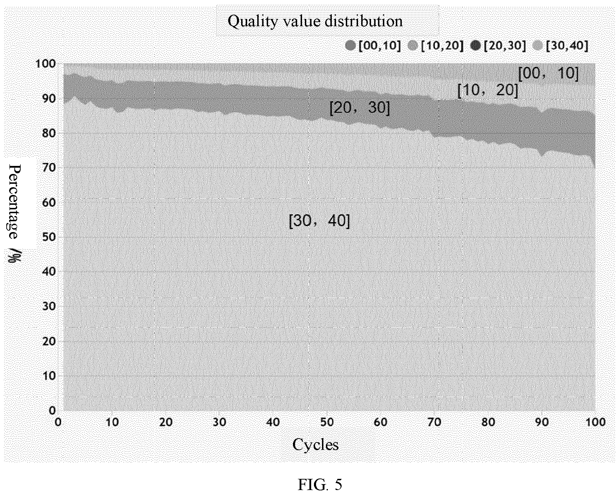

(1) Four first nucleotide analogs (see FIG. 1) and four second nucleotide analogs (see FIG. 2) were synthesized, blocking groups of the first nucleotide analogs were azido-methylene, and bases of the four first nucleotide analogs are adenine A, cytosine C, guanine G and thymine T, respectively; four markers were linked to bases of the four second nucleotide analogs by means of linkers (azido-methylene) respectively, with AF-532 linked to adenine A, IF-700 linked to cytosine C, CY5 linked to guanine G, and ROX linked to thymine T;
(2) Preparation of a sequencing reagent: polymerization solution 1: 50mM Tris-HCl, 50mM NaCl, 10mM (NH₄)₂SO₄, 0.02mg/mL polymerase 9N (Salus-bio), 3mM MgSO₄, 1mM EDTA, and the four first nucleotide analogs prepared above, each 1 µM;
   Polymerization solution 2: 50mM Tris-HCl, 50mM NaCl, 10mM (NH₄)₂SO₄, 0.02mg/mL polymerase 9N (Salus-bio), 3mM MgSO₄, 3mM CuCl₂, and the four first nucleotide analogs prepared above, each 1 µM;
   Elution buffer: 5× sodium citrate buffer (SSC), 0.05% Tween-20;
   Pre-wash buffer: 50mM Tris-HCl, 0.5mM NaCl, 10mM EDTA, 0.05% Tween-20;
   Excision buffer: 20mM tris(3-hydroxypropyl)phosphine (THPP), 0.5M NaCl, 50mM Tris-HCl, pH 9.0, 0.05% Tween-20;
   Imaging reaction solution: 1mM Tris-HCl, 40mM sodium L-ascorbate, 50mM NaCl, 0.05% Tween-2.
(3) Sequencing: the specific sequencing principle is shown in FIG. 3:
   Polymerization: a sequencing primer was hybridized onto a molecule to be detected to form a hybridized template strand and a primer strand; 400 µL of polymerization solution 1 was added to the chip amplified in part 1 above to bond the polymerase 9N to each DNA strand of the DNA library amplification cluster, and the temperature was set to 55°C for reaction for 1 min to polymerize the four first nucleotide analogs to a 3'-end of the primer strand under the action of the polymerase 9N, such that the primer strand was blocked and could not undergo polymerization; then, 200 µL of the elution buffer was added to wash away the four first nucleotide analogs that were incompletely reacted;
   Bonding: 400 µL of polymerization solution 2 was added, the temperature was set to 55°C for reaction for 1 min, the second nucleotide analogs formed a stable complex with the nucleic acid molecule to be detected (the template strand) and the first nucleotide analogs under the action of the polymerase 9N (containing aspartic acid) and metal ions Mg²⁺ and Cu²⁺, such that the second nucleotide analogs were stably chelated at the position of one nucleotide (the 3'-end of the primer strand) of the polymerization in the previous step; then, 200 µL of the elution buffer was added to wash away the second nucleotide analogs that were not bound to the 3'-end of the primer strand;
   Imaging: 200 µL of the imaging reaction solution was added, fluorescence signals of the whole chip were acquired through the optical system of the sequenator, signals of the bases in the primer strand were analyzed, and the bases at the corresponding positions were determined;
   Elution: 600 µL of the pre-wash buffer was added for reaction for 1 min to remove the metal ions non-covalently bonded with the second nucleotide analogs by reaction with the metal ions and release the second nucleotide analogs from the stable binding system;
   Excision: 200 µL of the excision reaction buffer was added, the temperature was set to 60°C for reaction for 1 min to remove the blocking group (azido-methylene), and then 200 µL of the elution buffer was added to repeat the washing once;
   The polymerization-excision process was repeated to perform a next sequencing cycle, and 100 bp sequencing was performed in total.
(4) Sequencing results: as shown in FIG. 4, because four fluorophores were used for distinguishing the four types of bases in the above sequencing process, A, G, C and T were used to represent signals of the four types of bases detected in the first cycle and the 100^{th} cycle respectively, wherein upper signal images in FIG. 4 correspond to the bases detected in the first cycle, lower signal images in FIG. 4 correspond to the bases detected in the 100^{th} cycle, and the grayscale values of bright points in the images indicate the intensity of the respective signals.
(5) Sequencing Q-values are shown in FIG. 5, wherein the Q-value represents the sequencing accuracy, the horizontal axis indicates the cycle number, and the vertical axis indicates the percentage of the data volumes for respective Q-values. The quality value Q [30,40] indicates the percentage of detected bases with an accuracy of over 99.9% in the current sequencing cycle, and it can be seen from FIG. 5 that more than 90% of detected bases have an accuracy of over 99.9% in each cycle, indicating that the sequencing method provided by the embodiment has a high accuracy.

## Claims

1. A gene sequencing method, **characterized by** comprising the following steps:
S1: hybridizing a sequencing primer onto a nucleic acid molecule to be detected to form a hybrid template strand and a primer strand;
S2: performing base pairing on a first nucleotide analog and the nucleic acid molecule to be detected, and linking the first nucleotide analog to the primer strand, wherein the first nucleotide analog has a blocking group;
S3: performing base pairing on a second nucleotide analog and the nucleic acid molecule to be detected, the second nucleotide analog forming a complex with the nucleic acid molecule to be detected and the first nucleotide analog under the action of metal ions and a polymerase, wherein the metal ions are bonded with coordinating atoms in the first nucleotide analog and coordinating atoms in the second nucleotide analog such that the second nucleotide analog is stably chelated at the 3'-end of the primer strand, and wherein the second nucleotide analog has a marker; wherein the chemical formula of the second nucleotide analog is as follows: 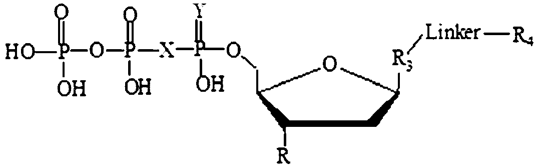
where, X and Y are each any one of oxygen (O), sulfur (S) and imino (NH), and X and Y are not both oxygen (O); R is any one of hydroxy (-OH), hydrogen (H), methoxyl (-OMe), amino (-NH₂) and sulfhydryl (-SH); R₃ is any one of A, G, C and T, Linker is a linker, and R₄ is the marker; wherein the metal ions are divalent metal ions, comprising at least one of Mg²⁺, Cu²⁺, Zn²⁺, Mn²⁺ and Ca²⁺;
S4: detecting the marker, and identifying a base in the nucleic acid molecule to be detected; and
S5: removing the blocking group and the second nucleotide analog, and repeating S2-S4 to perform a next cycle of sequencing.

2. The gene sequencing method according to Claim 1, **characterized in that** the blocking group is linked to a 3'-hydroxyl of the first nucleotide analog.

3. The gene sequencing method according to Claim 2, **characterized in that** the blocking group comprises at least one of azido-methylene, allyl, 2-nitrobenzene methyl and azoic compounds.

4. The gene sequencing method according to Claim 1, **characterized in that** the marker comprises at least one of Alexa Fluor, iFluor, cyanine, ROX and derivatives thereof.

5. The gene sequencing method according to Claim 1, **characterized in that** the linker comprises at least one of alkyl, allyl, azido-methylene, 2-nitrobenzyl and di-sulfhydryl.

6. The gene sequencing method according to Claim 1, **characterized in that** in step S4, detecting the marker comprises observing a solid phase of a double-stranded DNA formed by the template strand and the primer strand using a fluorescence microscope or an optical system of a sequenator, or performing fluorescence detection on a solution containing the double-stranded DNA using a fluorescence detector.

7. The gene sequencing method according to Claim **1, characterized in that** in step S5, a buffer containing a metal chelator is added to be bonded with and remove the metal ions so as to release the second nucleotide analog from the primer strand, and the metal chelator comprises at least one of ethylenediaminetetraacetic acid, ethylenediaminetetraacetate, nitrilotriacetic acid, citric acid, citrate, tartaric acid and gluconic acid.

8. The gene sequencing method according to Claim 1, **characterized in that** in step S5, the blocking group is removed by photocleavage or by adding an organic reagent, and the organic reagent comprises at least one of a sulfhydryl group reagent, an organic phosphine reagent and sodium hydrosulfite.

## Patentansprüche

1. Gensequenzierungsverfahren, **gekennzeichnet durch** die folgenden Schritte:
S1: Hybridisieren eines Sequenzierungs-Primers auf ein nachzuweisendes Nukleinsäuremolekül, um einen Hybrid-Templat-Strang und einen Primer-Strang zu bilden;
S2: Durchführen von Basenpaarung an einem ersten Nukleotidanalogon und dem nachzuweisenden Nukleinsäuremolekül und Verknüpfen des ersten Nukleotidanalogons mit dem Primer-Strang, wobei das erste Nukleotidanalogon eine Blockierungsgruppe aufweist;
S3: Durchführen von Basenpaarung an einem zweiten Nukleotidanalogonon und dem nachzuweisenden Nukleinsäuremolekül, wobei das zweite Nukleotidanalogonon unter Einwirkung von Metallionen und einer Polymerase einen Komplex mit dem nachzuweisenden Nukleinsäuremolekül und dem ersten Nukleotidanalogon bildet, wobei die Metallionen an koordinierende Atome in dem Nukleotidanalogon koordinierende Atome und dem zweiten Nukleotidanalogon gebunden werden, derart, dass das zweite Nukleotidanalogon am 3'-Ende des Primer-Strangs stabil komplexiert wird, und wobei das zweite Nukleotidanalogon einen Marker aufweist; wobei die chemische Formel des zweiten Nukleotidanalogons wie folgt ist: 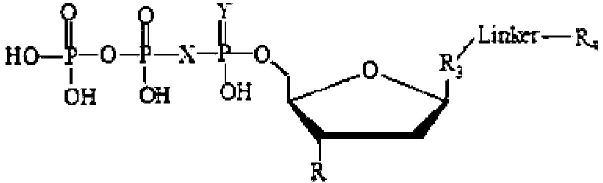
worin X und Y jeweils beliebige von Sauerstoff (O), Schwefe (S) und Imino (NH) sind und X und Yare nicht beide Sauerstoff (O) sind; R ein beliebiges von Hydroxy (-OH), Wasserstoff (H), Methoxyl (-OMe), Amino (-NH₂) und Sulfhydryl (-SH) ist; R₃ ein beliebiges von A, G, C und T ist, Linker ein Linker ist und R₄ der Marker ist; wobei die Metallionen zweiwertige Metallionen sind, die wenigstens eines von Mg²⁺, Cu²⁺, Zn²⁺, Mn²⁺ und Ca²⁺ umfassen;
S4: Nachweisen des Markers und Identifizieren einer Base in dem nachzuweisenden Nukleinsäuremolekül; und
S5: Entfernen der Blockierungsgruppe und des zweiten Nukleotidanalogons und Wiederholen von S2 bis S4, um einen nächsten Sequenzierungszyklus durchzuführen.

2. Gensequenzierungsverfahren nach Anspruch 1, **dadurch gekennzeichnet, dass** die Blockierungsgruppe mit einem 3'-Hydroxyl des ersten Nukleotidanalogons verknüpft wird.

3. Gensequenzierungsverfahren nach Anspruch 2, **dadurch gekennzeichnet, dass** die Blockierungsgruppe wenigstens eines von Azido-methylen, Allyl, 2-Nitrobenzolmethyl und Azoverbindungen umfasst.

4. Gensequenzierungsverfahren nach Anspruch 1, **dadurch gekennzeichnet, dass** der Marker wenigstens eines von Alexa Fluor, iFluor, Cyanin, ROX und Derivaten davon umfasst.

5. Gensequenzierungsverfahren nach Anspruch 1, **dadurch gekennzeichnet, dass** der Linker wenigstens eines von Alkyl, Allyl, Azido-methylen, 2-Nitrobenzyl und Di-sulfhydryl umfasst.

6. Gensequenzierungsverfahren nach Anspruch 1, **dadurch gekennzeichnet, dass** in Schritt S4 das Nachweisen des Markers Beobachten einer Festphase einer doppelsträngigen DNA, die durch den Templat-Strang und den Primer-Strang gebildet wird, unter Verwendung eines Fluoreszenzmikroskops oder eines optische Systems, oder Durchführen von Fluoreszenznachweis an einer Lösung, die die doppelsträngige DNA enthält, unter Verwendung eines Fluoreszenzdetektors umfasst.

7. Gensequenzierungsverfahren nach Anspruch 1, **dadurch gekennzeichnet, dass** in Schritt S5 ein Puffer, der einen Metallchelator enthält, zugesetzt wird, um sich an die Metallionen zu binden und diese zu entfernen, um das zweite Nukleotidanalogon aus dem Primer-Strang freizusetzen, wobei der Metallchelator wenigstens eine von Ethylendiamintetraessigsäure, Ethylendiamintetraacetat, Nitrilotriessigsäure, Zitronensäure, Citrat, Weinsäure und Gluconsäure umfasst.

8. Gensequenzierungsverfahren nach Anspruch 1, **dadurch gekennzeichnet, dass** in Schritt S5 die Blockierungsgruppe durch Photolysespaltung oder durch Zusetzen eines organischen Reagens entfernt wird, wobei das organische Reagens wenigstens eines von einem Sulfhydrylgruppen-Reagens, einem organischen Phosphin-Reagens und Natriumhydrosulfit umfasst.

## Revendications

1. Procédé de séquençage génétique, **caractérisé en ce qu'**il comprend les étapes suivantes :
S1 : hybrider une amorce de séquençage sur une molécule d'acide nucléique à détecter pour former un brin hybride et un brin d'amorce ;
S2 : effectuer l'appariement de bases sur un premier analogue nucléotidique et la molécule d'acide nucléique à détecter, et relier le premier analogue nucléotidique au brin d'amorce, dans lequel le premier analogue nucléotidique possède un groupe bloquant ;
S3 : effectuer l'appariement de bases sur un deuxième analogue nucléotidique et la molécule d'acide nucléique à détecter, le deuxième analogue nucléotidique formant un complexe avec la molécule d'acide nucléique à détecter et le premier analogue nucléotidique sous l'action d'ions métalliques et d'une polymérase, dans lequel les ions métalliques sont liés aux atomes de coordination du premier analogue nucléotidique et aux atomes coordonnés dans le deuxième analogue nucléotidique de sorte que le deuxième analogue nucléotidique soit chélaté de manière stable à l'extrémité 3' - du brin amorce, et dans lequel le deuxième analogue nucléotidique possède un marqueur ; dans lequel la formule chimique du deuxième analogue nucléotide est la suivante : 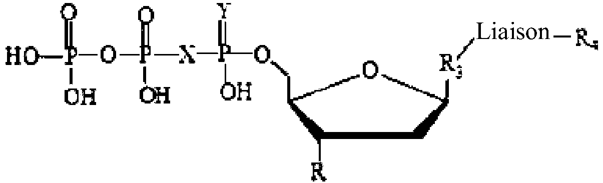
dans lequel X et Y sont chacun un composé d'oxygène (O), de soufre (S) et d'imino (NH), et X et Y ne sont pas tous deux de l'oxygène (O) ; R est l'un des deux entre hydroxyle (-OH), hydrogène (H), méthoxyle (-OMe), amino(-NH2) et sulfhydryle (-SH) ; R₃ est l'un de A, G, C et T, la Liaison est une liaison, et R₄ est le marqueur ; dans lequel les ions métalliques sont des ions métalliques divalents, comprenant au moins un de Mg²⁺, Cu²⁺, Zn²⁺, Mn²⁺ et Ca²⁺ ;
S4 : détecter le marqueur et identifier une base dans la molécule d'acide nucléique à détecter ; et
S5 : retirer le groupe bloquant et le deuxième analogue nucléotide, et répéter S2-S4 pour effectuer un cycle suivant de séquençage.

2. Procédé de séquençage génétique selon la revendication 1, **caractérisé en ce que** le groupe bloquant est lié à un 3'-hydroxyle du premier analogue nucléotide.

3. Procédé de séquençage génétique selon la revendication 2, **caractérisé en ce que** le groupe bloquant comprend au moins un composé d'azido-méthylène, d'allyle, de 2-nitrobenzène méthyl et de composés azoïques.

4. Procédé de séquençage génétique selon la revendication 1, **caractérisé en ce que** le marqueur comprend au moins un Alexa Fluor, iFluor, cyanine, ROX et leurs dérivés.

5. Procédé de séquençage génétique selon la revendication 1, **caractérisé en ce que** la liaison comprend au moins un alkyle, allyle, azido-méthylène, 2-nitrobenzyle et di-sulfhydryle.

6. Procédé de séquençage génétique selon la revendication 1, **caractérisé en ce que**, à l'étape S4, la détection du marqueur consiste à observer une phase solide d'un ADN double brin formé par le brin modèle et le brin amorce à l'aide d'un microscope à fluorescence ou un système optique de séquençage, ou effectuer la détection de fluorescence sur une solution contenant l'ADN double brin à l'aide d'un détecteur de fluorescence.

7. Procédé de séquençage génétique selon la revendication 1, **caractérisé en ce qu'**à l'étape S5, un tampon contenant un chélateur métallique est ajouté pour être lié et retirer les ions métalliques afin de libérer le deuxième analogue nucléotidique du brin d'amorce, et le chélateur métallique comprend au moins un acide éthylénédiaminetétraacétique, éthylénédiaminetétraacétate, acide nitrilotriacétique, acide citrique, citrate, acide tartrique et acide gluconique.

8. Procédé de séquençage génétique selon la revendication 1, **caractérisé en ce qu'**à l'étape S5, le groupe bloquant est retiré par photoclivage ou par ajout d'un réactif organique, et le réactif organique comprend au moins un réactif du groupe sulfhydryle, un réactif organique de phosphine et un hydrosulfite de sodium.
